# EUROPEAN PATENT APPLICATION

(11) **EP 1 479 688 A1**
(43) Date of publication of application: **24.11.2004**
(21) Application number: 03011713.9
(22) Date of filing: 23.05.2003
(51) Int. Cl.: C07K 1/04

(54) **Solid-phase peptide synthesis using non-covalent attachment of the growing peptide chain**

(71) Applicant: AplaGen GmbH, 52499 Baesweiler (DE)
(72) Inventor: The designation of the inventor has not yet been filed
(74) Representative: Meyers, Hans-Wilhelm, Dr.Dipl.-Chem.

(57) **Abstract**

A method for solid phase synthesis of peptides, characterized by a non-covalent attachment of the growing peptide chain to the solid phase during the synthesis cycles.

## Description

### State of the Art

Chemical synthesis of peptides is well established. In principal, two different methods can be distinguished. The synthesis in solution is often very time consuming and therefore not useful for scientific research, whereas the synthesis on a solid support allows a fast optimization of reaction cycles. The protocols available for solid phase peptide synthesis (SPPS) are based on the initial technique developed by Dr. Robert Bruce Merrifield (Merrifield, R.B. (1963), J. Amer. Chem. Soc., 85, 2149), who had the idea to synthesize peptides with defined sequences on an insoluble solid support which has the benefit that separation of soluble reagents can easily be handled by simple filtration. The whole process can be automatized on suitable machinery. The SP peptide synthesis has developed extremely fast. Nowadays it is known in a number of variants (summarized here as Merrifield-type synthesis) and consists of the following steps:
1. In a first step, a suitable solid support matrix (resin) is chosen, to which an amino acid derivative is fixed covalently through its C-term. Usually the first C-terminal amino acid of the sequence to be synthesized is anchored to the solid support by the help of a linker. The amino and side chain functions of this first amino acid are protected by protective groups according to the state of the art, usually compatible with Fmoc- or Boc- chemistry.
2. In a second step, the group protecting the N-term of the first amino acid is selectively removed.
3. In a 3^{rd} step, the next amino acid derivative corresponding to the 2^{nd} amino acid in the sequence to be synthesized is coupled to the free terminal amino group of the first amino acid, while themselves having the amino and side chain function protected by the appropriate protecting groups.
4. In the 4^{th} step, the N-term-protecting group is removed from the freshly coupled amino acid derivative and step 3 is repeated for the next amino acid.

Thus, solid phase synthesis of peptides includes a cyclic process by which a defined sequence of amino acids can be built on a solid phase support. At the end of the process, the peptide is released from the solid support e.g. by treatment with trifluoroacetic acid (including different scavengers). Simultaneously, the protecting groups which might have been present at side chains of the amino acids, thereby protecting imidazole-, thiol-, carboxy-, amino-, alcohol- or other functionally relevant groups, might be removed. This leads to the final peptide, which often needs to be purified on suitable chromatographic equipment, usually an LC/MS System, which is able to simultaneously separate and analyse the components. These and further steps are considered in depth in standard textbooks on peptide synthesis (e.g. "Principles of Peptide Synthesis (Springer Laboratory)" or "Amino Acid and Peptide Synthesis (Oxford Chemistry Primers, 7)").

While synthesis of small peptides (up to 30meres) is usually no problem with SPPS techniques, there are limitations with peptides at a size of 40 up to 120 (or even more) amino acids. Their properties correspond much better to the term "small protein" than to the term "large peptide":
a) Peptides of this size usually have not only the primary structure (sequence), but also a secondary structure (e.g. helix, beta-sheet) and a tertiary structure (e.g. leucine-zipper, disulfide bridging of domains) to be constructed. While the various variants of the SPPS technique aim solely at the build up of the primary structure, they do not provide any tool to control the intramolecular formation of secondary and tertiary structures in a suitable way. The lack of control mechanisms on the folding of larger peptides is even aggravated by the fact that the simultaneous detachment of all products from the resin and removal of protective groups leads to very high local product concentrations. Under these conditions not only intramolecular folding occurs. In many cases, the small proteins released from the resin tend to interact with each other instead of intramolecular folding. Similar problems occur, when the lyophilized fractions containing the purified product eluted from the LC-system are reconstituted with solvent. Thus, intramolecular folding and intermolecular interactions compete and there is no technique available to control this process adequately. This often results in functionally useless multimolecular aggregates, which do not show any desired biological activity.
b) In case of the synthesis of large peptides, it is preferred to use appropriately modified (protected, partially protected or unprotected) peptide fragments instead of single amino acid residues for each cycle of synthesis. Usually, small fragments are either connected by ligation or fragment condensation techniques in order to minimize the number of synthetic steps. This is due to the fact that the cycles usually do not have quantitative yields and too many cycles often end in an unacceptable decline of yield. Moreover problematic couplings of single amino acids simply can be prevented by coupling a whole fragment instead of a single amino acid. In the case of fragment condensation the problem arises, that rather long coupling times have to be used during each coupling cycle. The low reaction velocity is diffusion-limited and due to the non-diffusion of the fixed peptide chain and suboptimal diffusion behaviour of the fragments in the pores of the resin surrounding. The long reaction times often lead to a considerable degree of racemisation at the C-terminal amino acid of the fragments to be coupled, which defines a need to keep reaction times as short as possible. The best way to overcome this problem is by coupling in solution. The classical SPPS does not provide an approach to bring both reaction partners in solution and reattach them during the next step of the cycle, while repeating this procedure at each step of the synthesis.
c) It is often difficult to control the desired density of starting residues on a polymeric support and the loading of the resin with the first amino acid often goes along with racemization. Though many preloaded resins are commercially available, these do not cover all loadings and the best value for a given peptide synthesis problem often is hard to find.

While metal affinity was not in use in the field of chemical synthesis of peptides, metal affinity is known in the area of recombinant production of biomolecules. However, it is used as a tool for purification of molecules from crude mixtures in a single step. Patents on such strategies using Agarose-derivatives containing Iminodiacetate and Nitrilotriacetate exist (EP 0253303B1; US 4,877,830). Beaded Agarose suited for chromatographic purification of recombinant products is commercially available. Moreover, peptides containing metal-affinity side chains were constructed with the intention to use the side-chain fixed transition metal complexes as luminescent labelling reagents for a given peptide (e.g. WO 9603651A1; EPA 0178450 A2). All these descriptions document the applicability of metal chelate techniques in the analysis and/or purification of biomolecules. However, they do not imply the nature of the invention and its applications as described below.

### Characterisation of the Invention

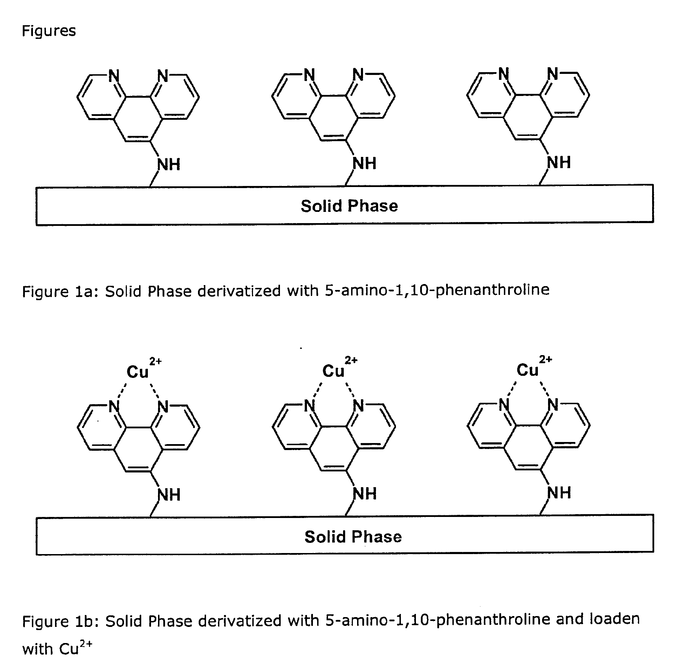

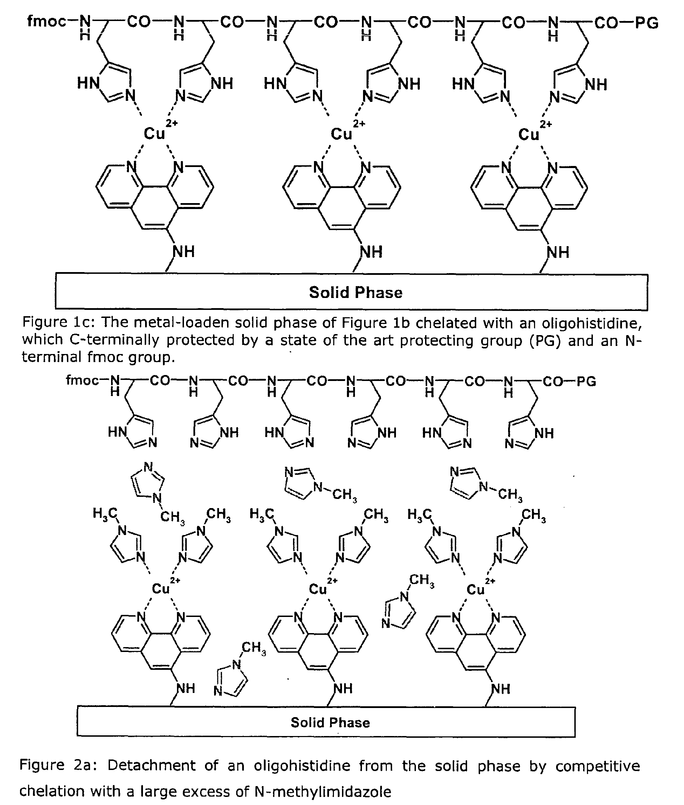

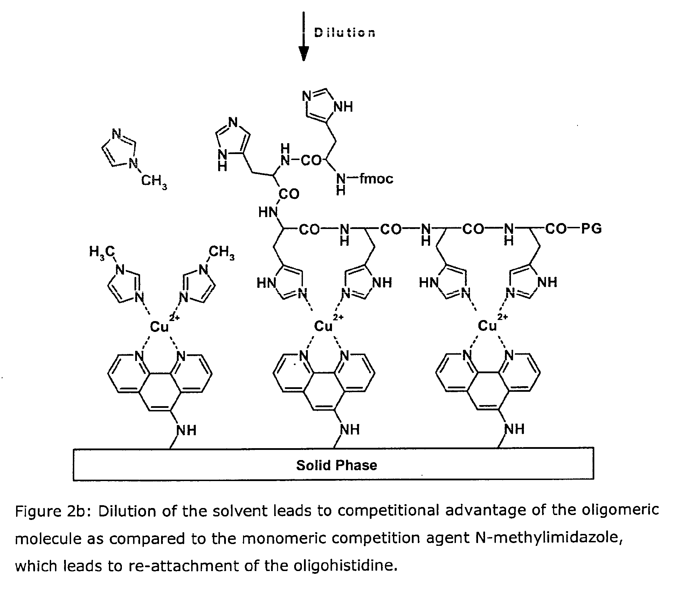

The invention presented here overcomes the disadvantages of the actual state of the art using the common principle of the invention:
Instead of covalent attachment to a suitable resin the invention relies on metal complexes which fix the growing peptide structure to an appropriate solid support or resin. Such metal complexes can be used during the repetitive synthesis steps and allow to detach the growing peptide chain during the coupling step, while it can be attached again before the following steps. Moreover, the same principle of anchoring the peptide chain reversibly to a solid support can be used to control folding of the purified product. For this purpose, the product is purified and reattached to a metal affinity resin at a suitably diluted relation of number of product molecules to resin surface. If chosen correctly, this will statistically avoid that product molecules can meet each other and thus lead to a preference for intramolecular instead of intermolecular folding (aggregation). Having been attached, the product is then treated with a number of solvents which allow a gradual folding of the molecule and support the correct intramolecular configuration of the peptide chains. The folding steps can be sealed by the formation of e.g. disulfide bonds in the correct position. At the end of this procedure the stabilized product is released correctly folded into - usually aqueous - surrounding. This is achieved by correct use of the various components of the invention, namely
   a) A resin, which was derivatized with a suitable ligand being able to complex with divalent cations, preferably Cu²⁺, Ni²⁺, Co²⁺, Zⁿ²⁺, Mg²⁺ or Ca²⁺, although other ions might be suitable, too. In a preferred version the invention uses ligands suitable for Cu²⁺, Ni²⁺ or Co²⁺. Examples for suitable ligands are 5-amino-1,10-phenanthroline, triphenylphospine or 6-aminopurine. The ligand has to be compatible with the reagents used during peptide synthesis, especially if Fmoc-chemistry is used. For this reason common ligands like di- or trinitriloacetic acid as well as iminodiacetic acid are not suitable. The free carboxy groups of these common ligands would interfere with the coupling reagents used during peptide synthesis. If complexed according to the principle of the invention, the resin surface consists of the ligand molecules complexed with the divalent cations. The divalent cation is complexed such that free valencies for additional complexing are available (see Fig. 1).
   b) Using a starting point for synthesis, which is compatible with peptide synthesis and can be used to anchor the growing peptide chain to the metal affinity surface on the solid support. Molecule(s) having this property are characterized by a (protected or unprotected) terminal amino group and consist of one or several residues, which are able to complex with the free valencies of the complexed metal ions at the resin surface. In a preferred version of the invention the starting point is defined by an oligohistidine, formed by at least 2 serial (L- or D-)histidine residues, preferably by 6 (L- or D-) histidine residues. Both, the carboxy and aminoterminus of the oligohistidine may be protected by appropriate protective groups; the aminoterminus may preferably be protected by Fmoc chemistry (see Fig. 2). The starting point can be used as pure N- and C-terminally protected oligohistidine. However, it is easily possible and part of the invention to use extended versions of the oligohistidine as starting point for synthesis. Thus, it can be useful to extend the oligohistidine by short amino acid sequences, which code for protease recognition sites. This would allow selective removal of the oligohistidine after the synthesis process and the folding protocol. In another modification of the invention a sequence is added, which allows simple recognition of the final peptide chain by an antibody (a tag-sequence, e.g. a myc-tag or others). Moreover, addition of a biotinylated amino acid (e.g. biotinylated (D-, L-) lysine) preferably at the C-terminus of the oligohistidine would allow to establish simple quantification based on the specific interactions between biotin and avidin-like molecules.
   c) While the metal-affinity resin can always be prepared by maximal load with ligand and complexed ion, the density of starting points for peptide synthesis can be controlled very easily: A suitable starting point like an oligohistidine will attach quantitatively to the resin and the load can be determined by the amount of starting points added to the resin during the 1^{st} attachment step. Since complexation reactions are usually fast, attachment can be completed in organic or aqueous solvent within a few minutes.
   d) Having been synthesized, detached from the metal-affinity resin and purified by e.g. conventional Liquid Chromatography/Mass Spectrometry (LC/MS), the product can be subjected to a final step of the synthesis, a refolding protocol. For this protocol, the product has to be dissolved in monomolecular solution in a suitable solvent. Usually, the most preferred solvent is the elution solvent from the LC-purification system, although the product can be lyophilized and reconstituted in a suitable solvent. From this monomolecular solution the product is reattached to a metal affinity surface. This can be one of the resins described above under a), but for this purpose the use of resins based on iminodiacetic acid or trinitriloacetic acids is also included. This is possible since refolding usually does not use reagents, which activate free carboxylic groups. Density of attachment is chosen very low in order to avoid that product molecules come into close contact with each other. After completion of attachment a series of solvents is passed gradually along the resin. In principle the first solvents are highly denaturing and/or chaotropic in order to bring all product molecules in a comparable state of folding. Gradually, the next steps and solvents will approach physiological conditions in such a way that molecular folding is supported. At the end of the protocol, the product molecules are in the desired - usually aqueous - final solvent, might be finally sealed with e.g. a disulfide bond and can be released from the resin.
   e) While attachment of a suitable starting point for peptide synthesis (e.g. a oligohistidine) is achieved by adding a slightly alkaline or neutral solution of the oligohistidine to a metal-affinity resin, detachment can be achieved by different techniques:
      In both, organic solvents and aqueous solvents, detachment can be achieved by increasing acidity of the solvent to a critical degree (in aqueous media a pH of 5, at least below 6 is sufficient). A very smooth and in most cases preferred principle is competitive detachment in the presence of a competitive ligand for the metal-affinity resin. As a principle of the invention, the competing ligand should have a comparable affinity to the metal-affinity resin than the single residues of the - usually oligomeric - starting point to be used. In a preferred embodiment of the invention, which uses an oligohistidine as starting point, the competing agents are imidazole (in aqueous solvents) or N-methyl imidazole (in organic solvents). Detachment is achieved by adding a large excess of competing agent to the solvent. In case of aqueous solvents 250mM solutions of imidazole would suffice. Re-attachment can be achieved by dilution of the solvent containing the competing agent, usually by a factor ~10-20. This leads to a drop in the concentration of competing agent and prefers re-attachment of the oligomeric starting point to the support. Using the latter principle, it is possible to repetitively detach and attach the growing peptide chain from and onto the affinity matrix. In a preferred embodiment of the invention, detachment is achieved during the coupling step of peptide synthesis, while reattachment is induced by dilution of the reaction mixture prior to the washing steps preceding the following deprotection. Using these steps, it is possible to use "transient" liquid phase synthesis and its advantages, especially for synthesis of large peptides by fragment condensation approaches.

Another advantage associated with the invention is that metal-affinity resins can be reused after peptide synthesis. This can be achieved by removing the divalent cation and reloading the resin with the same or another appropriate cation. Since the binding strength of the various cations is different from each other, a method of modifiying the attachment and re-attachment procedures and the stability of the resulting complexes is to choose for another cation as bridging ligand between the resin surface and the starting point of the synthesis.

By introducing a new non-covalent principle of attachment to a solid phase support, the invention provides advantages during peptide synthesis itself, but also for post-synthetic refolding operations, which might be necessary in the case of products with a defined tertiary structure.

### Examples

### Example 1:

### Binding 5-amino-1,10-phenanthroline to 2-chlorotrityl resin

1g 2-chlorotrityl chloride resin (1.3mmol/g; 200-400 mesh) is suspended in 8ml dry THF and a suspension of 0.4mmol (780mg) of 5-Amino-1,10-phenanthroline and 0.8mmol DIEA is added to this mixture, which is then shaken for 12 hours. The loaded resin is then washed 20 times with 20ml DMF. Afterwards 20ml of DCM/MeOH/DIEA (80/15/5) is added for 30 minutes. The resin is washed 6 times with DMF and stored under DMF.

### Example 2:

### Binding a metal cation to the resin, including washing

20ml of a satured solution of Cu(SO₄)₂ in DMF are added to the resin and left on a vortexer for 30 minutes. The resin is washed with DMF until the DMF stays colorless. To remove adsorbed Cu²⁺ the resin is then washed by repetetive washing steps including at least 2 washings with 0.25mol/l 1-methyl-imidazole.

### Example 3:

### Attaching an oligohistidine to the surface

20ml of an oligohistidine solution (1mg/ml DMF) are added to the resin and the mixture left on a vortexer. After 30 minutes the reaction is monitored by HPLC (injection of 50 µl on analytical C18-column with gradient from 10-100% Acetonitrile in 30 minutes). In most cases the reaction is completed after 30 minutes and no more oligohistidine can be detected in solution. The resin is then washed 6 times with DMF and stored under DMF.

### Example 4:

### Detaching an oligohistidine from the surface

The loaded resin is treated with 20ml of 1-methyl-imidazole in DMF (0.25mol/l) and left on a vortexer for 10 minutes. The detached oligohistidine is isolated by removing the volatile components in vacuo.

### Example 5

### Deprotection of the oligohistidine and completion of a synthesis cycle

20ml Piperidine (25% solution in DMF) are added to the resin and left on a vortexer for 20 minutes. The resin is washed six times with 20ml DMF. Then a solution of 5mmol Fmoc-Ala-OH in 5ml DMF is prepared and 7.5mmol HOBt and 5mmol DIEA is added. After 5 minutes on the vortexer, 5mmol of PyBOP and 5mmol DIEA is added to the solution and this mixture is poored onto the resin. After 60 minutes the coupling is finished and the resin washed 6 times with 20ml DMF. The resin can be washed with DCM and dried in vacuo or it can be directly used for the next synthesis cycle.

## Claims

1. A method for solid phase synthesis of peptides, **characterized by** a non-covalent attachment of the growing peptide chain to the solid phase during the synthesis cycles.

2. A method for solid phase synthesis of peptides according to claim 1, **characterized by** a metal complex structure, which anchors the growing peptide chain to the solid phase during the synthesis cycles.

3. A method for solid phase synthesis of peptides according to claim 1 and 2, in which the metal complex structure consists of
a) ligands, which are covalently fixed to the solid phase
b) metal ions (Meⁿ⁺;n between 1 and 3, preferably 2), which are complexed by the ligands, while free coordination sites still remain available
c) single or oligomeric amino acids containing side chains, which are able to complex with the free coordination sites of the partially saturated Meⁿ⁺ ions described under b).

4. A method for the solid phase synthesis of peptides according to claims 1-3, in which the solid phase is **characterized by** the presence of functional chemical groups preferably being selected from amino-, heterocyclic nitrogen-, carboxy-, hydroxyl-, thiolgroups or other functional entities for which coupling reactions with suitable ligands are available.

5. A method for the solid phase synthesis of peptides according to claims 1-4, in which the ligands contain a functional group which can be coupled chemically to the functional chemical groups at the surface of a solid phase according to claim 4, and, in which the same ligands contain one or more functional groups being able to complex metal ions (Meⁿ⁺), preferably being selected from heterocyclic nitrogen, aza groups, carboxy groups, sulphur- or phosphorus containing chelating groups.

6. A method for the solid phase synthesis of peptides according to claim 1-5, in which metal ions (Meⁿ⁺) are complexed with ligands onto a solid phase; preferably, but not exclusively these metal ions being selected from Ni²⁺, Cu²⁺, Co²⁺ or Zⁿ²⁺.

7. A method for the solid phase synthesis of peptides according to claim 1-6, in which mono- or oligomeric amino acids with side chains being able to complex metal ions (Meⁿ⁺) are complexed to the free coordination sites of ligand/Meⁿ⁺ complexes, which are bound on a solid phase support.

8. A method for the solid phase synthesis of peptides according to claim 1-7, in which a Merrifield-type sequential reaction is implemented and used in order to attach additional mono- or oligomeric amino acids to the C- or N-terminus of the first mono- or oligomeric amino acids, which were bound by a metal complex to the solid support, whereby the appropriately protected amino acid derivatives or oligomeric fragments being attached in each cycle can be chosen or composed freely from any natural or unnatural amino acid.

9. A method for the solid phase synthesis of peptides according to claim 1-8, in which a competitive chelation agent is added to the coupling step of the Merrifield-type reaction schedule in order to detach the growing peptide chain from the solid support during that step, whereby suitable competitive chelation agents have about the same affinity constant as the individual side chains of the complexing oligomeric amino acids used to fix the growing peptide chain to the solid support.

10. A method for the solid phase synthesis of peptides according to claim 9, in which the reaction mixture of the coupling step containing the competitive chelation agent is diluted prior to the following washing steps in order to reattach the oligomeric amino acids containing side chains able to complex divalent metal cations - and thus the growing peptide chain - to the solid support.

11. A method for the solid phase synthesis of peptides according to claim 1-10, in which the final purified product still containing the mono-or oligomeric amino acids with side chains capable of forming complexes with divalent metal ions is processed in a second solid phase synthesis and for that purpose brought into monomolecular solution, preferably in the presence of chaotropic agents. Afterwards, the product is reattached at suitable dilution to a solid phase according to claims 4-6.

12. A method for the solid phase synthesis of peptides according to ciaim ii, in which the reattached product is exposed to solvents, which stepwise reduce chaotropy and support correct intramolecular folding of the product.

13. A method for the solid phase synthesis of peptides according to claim 11 and 12, in which - after solid-phase controlled refolding of the product - additional covalent modifications are introduced, which stabilize the refolded intramolecular structure. Preferably, but not exclusively, these covalent modifications are disulphide bridges or covalent links between amino acid side chains.

14. A method for the solid phase synthesis of peptides according to claim 4, in which the solid phase is chosen from a chlortrityl resin, from carboxylated melamine particles, or from carboxylated polyvinylalcohol polymeric support.

15. A method for the solid phase synthesis of peptides according to claim 4 and 14, in which the resin matrix contains ferromagnetic particles and allows the application of magnetic particle separation technology.

16. A method for the solid phase synthesis of peptides according to claim 7, in which the mono- or oligomeric amino acids contain imidazole side chains. Preferably, two or more histidine residues are used; most preferably, 6-10 histidine residues are used.

17. A method for solid phase synthesis of peptides according to claim 16, in which the oligohistidine used was extended at the N-terminus by a short amino acid sequence providing a specific protease recognition site. This is to enable enzymatic removal of the oligohistidine at the end of synthesis.

18. A method for solid phase synthesis of peptides according to claim 16 and 17, in which the modified or unmodified (according to claim 16) oligohistidine is extended at the C-terminus by one or more amino acids, which allow detection by detection systems according to the state of the art. Preferably, but not exclusively, a biotinylated amino acid can be attached to the C-terminus.

19. A method for solid phase synthesis of peptides according to claim 9 and 10, 16-18, in which the competitive chelation agent is imidazole or N-methylimidazole.

20. A method for solid phase synthesis of peptides according to claim 5, in which the ligand is chosen from derivatives of Iminodiacetic acid, Nitrilotracetic acid, phenanthroline, triphenylphosphine or 6-aminopurine. In a preferred embodiment, the ligand is chosen from 5-amino-1,10-phenanthroline or 6-aminopurine.

21. A method for solid phase synthesis of peptides according to claim 1-20, which is fully automated, compatible with synthesis roboters and in which separation of liquid and solid phase during synthesis cycles can be achieved by any state of the art technology preferably by sieving (size-based separation), centrifugation or magnetic particle separation technology.

22. A machine or device being able to carry out a solid phase synthesis of peptides according to claim 1-21.
